# EUROPEAN PATENT APPLICATION

(11) **EP 1 080 693 A1**
(43) Date of publication of application: **07.03.2001**
(21) Application number: 99116824.6
(22) Date of filing: 02.09.1999
(51) Int. Cl.: A61B 17/82, A61B 17/04

(54) **Haemostatic and/or reinforcing surgical suture system for sternal cerclages**

(71) Applicant: Zurbrügg, Heinz Robert, Dr. med., CH-3047 Bremgarten (CH)
(72) Inventor: Zurbrügg, Heinz Robert, Dr. med., CH-3047 Bremgarten (CH)
(74) Representative: HOFFMANN - EITLE

(57) **Abstract**

A haemostatic and/or reinforcing surgical suture system for closing an opened sternum following a mid-line thoracotomy with cerclages comprises a length of first surgical suture having respective ends and a needle disposed at each end of the first surgical suture. The haemostatic and/or reinforcing surgical suture system for sternal cerclages further comprises at least one length of second surgical suture connected to the first surgical suture in the vicinity of at least one end of said first surgical suture and/or at least one first puncture closure connected to the first and/or second surgical suture.

## Description

### Technical Field

The invention relates to a surgical suture system for cerclages, i.e. a loop-type application, for closure of the sternum following mid-line thoracotomy.

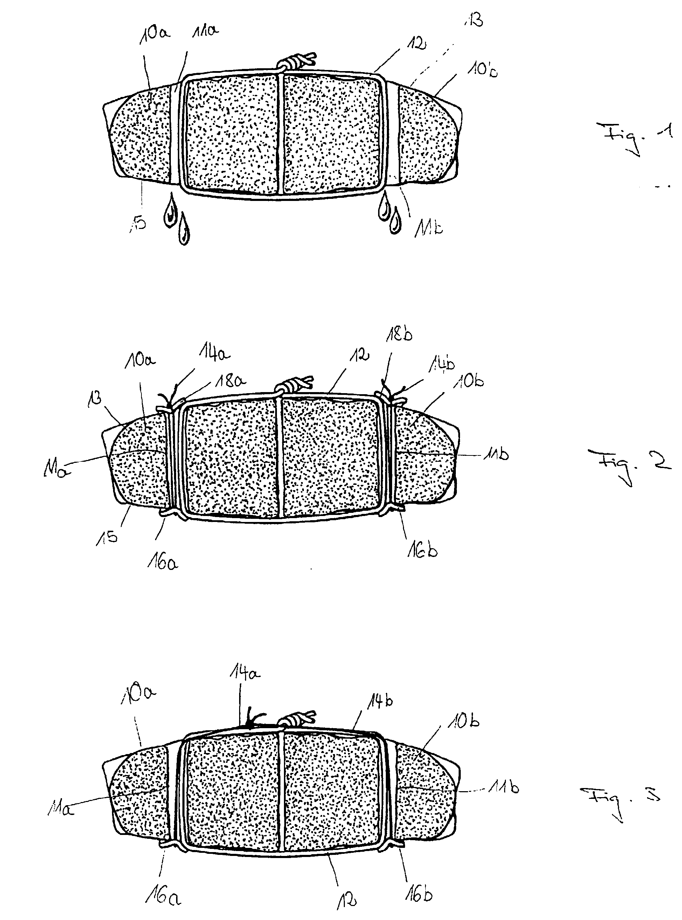

Haemostatic treatment in surgery, in particular in heart surgery, is becoming more and more important. The conventional sternal closure, following heart surgery through a mid-line thoracotomy, is performed using wires made of surgical steel which are passed through the sternal cortical layers 10a, 10b. Both ends of the surgical wires are tightly twisted together thus forming a cerclage. Therefore, the wire tends to incise into the sternal cortical layers (Fig. 1). The puncture channel becomes enlarged which leads to an oozing haemorrhage. Especially, at the posterior side of the sternum, this oozing haemorrhage is not accessible by conventional surgical haemostatic treatment (coagulation, suture of the source of the haemorrhage with a thread) and incision of the wire into the cortical layers of the sternum cannot be prevented.

### Prior Art

A sternal wire according to the prior art comprises a length of surgical suture. This surgical suture is usually made of wires, for example non-absorbable steel wires in accordance with German indurstrial standard DIN 1.4441, or in the case the sternal closure is used for children the suture material is often absorbable material. DIN 1.4441 steel is a high grade stainless steel alloy which consists chiefly of iron, chromium, nickel and molybdenum. A needle is disposed at one end of the surgical suture. The needle is, for example, an integrally hardened, atraumatic needle.

Usually six to eight wires are used for an adult. Each of the wires has a diameter of about 0,75 mm to 0,85 mm and a length of about 450 mm. Each of the needles has a half-circle shape with a diameter of about 49 mm and a cutting portion at one end.

When closing a sternum bisected by mid-line thoracotomy, the cutting needle with the wire is advanced, for example, first through the left half part of the sternum from anterior to posterior and then through the right half from posterior to anterior. This is done for all six to eight wires needed for closing the sternum. Afterwards the needles are separated from the wires and the two ends of each of the wires are tightened and connected to each other by twisting them, thus forming a cerclage. To avoid any invisible loops at the dorsal side of the sternal plate, the two ends of the wires protruding on the anterior side of the sternum are pulled alternately before connecting them. By performing this movement the surgeon can detect any loops on the invisible posterior side of the sternum, which would render the sternal closure weak.

To avoid sternal instability which is a major post-operative complication, a reinforced sternal closure system is known in the prior art. In addition to the wires crimped with the needles the system further comprises a staple gun and reinforcing staples. The staples, which are placed by the gun directly into the sternum, transfer the load from the sternal cerclage to a larger area of the cortical layer. This prevents the wire from cutting into the cortical layers of the sternum. However, these staples are only applicable on the anterior side of the sternum.

### Object of the Invention

The invention has the object to provide a surgical suture system for sternal cerclages and a sternal closure kit which are easily applicable in connection with haemostatic or haemostyptic treatment and with or without reinforcing effect. The term haemostatic is used throughout the description of the present invention, although the invention is not intended to be limited to haemostatic treatment and may also include haemostyptic treatment.

This object is solved by a sternal haemostatic and/or reinforcing surgical suture system for sternal cerclages according to claim 1 or 8 and a sternal closure kit according to claim 21. A puncture closure for the sternal closure kit is characterized by claim 23.

The invention is based on the idea to provide a needle on both ends of a surgical suture adapted for use as sternal cerclage. This offers the possibility to advance each needle and the surgical suture connected to it through the associated sternal half in the direction from the posterior to the anterior cortical layer. In particular, this means that the first needle is passed through, for example, the left sternal half and the right needle is passed through the right sternal half in the direction from the posterior to the anterior side of the sternum.

Therefore, a haemostatic and/or reinforcing surgical suture system for sternal cerclages comprising a length of surgical wire and a needle disposed at each end of the surgical wire offers the possibility to also provide puncture closures (seals), with or without the use of second surgical suture material, for closing the puncture channels as a part of a haemostatic and/or reinforcing treatment as both sternal cortical layers are cut in the same direction.

It has to be said that with a conventional sternal closure, possibly comprising a reinforcement, the cutting of the wires into the cortical layers of the sternum cannot be prevented totally. Moreover, the needle has always a diameter bigger than that of the wire. Therefore, the opening of the puncture channel is always bigger and oozing blood loss can only be prevented with a haemostatic sternal closure. The conventional sternal wire, i.e. a surgical suture crimped with a needle at only one end of the suture, does not offer the possibility to apply additional puncture closures (seals) and/or reinforcements onto the posterior side of the sternum while closing the sternum following mid-line thoracotomy. A haemostatic and/or reinforcing surgical suture system for sternal cerclages according to claim 1 is particularely simple in use and, additionally, represents the basis for reducing the loss of blood which, with the conventional method, is about 200 ml more. With a conventional sternal suture system it may occur that the needle becomes bent and dull upon advancing it through the first sternal half. This happens about 30 to 40% of the time, especially if a sternum is very strong. In this case, the needle has to be bent back as close as is practical to its original shape before advancing it through the second sternal half. Otherwise, the needle may become jammed in the sternum and has to be moved further by applying extra force. This results in an undesirably increasing the size of and/or tearing the edge of the puncture channel and, therefore, producing a higher blood loss and a diminished healing. These problems are advabtageously overcome with the sternal suture system according the present invention comprising needles at both ends.

Advantageous embodiments are characterised by dependent claims 2 to 7, 9 to 20, 22 and 24 to 31.

Preferably, the sternal cerclage according to the present invention is made of absorbable or non-absorbable material, in particular the surgical suture may be absorbable or non-absorbable. In this context, the term resorbable material is also often used, meaning a material which can be absorbed by the body. Throughout the description of the present invention these terms are used synonymously. Non-absorbable material, in particular wire, is used for closing an opened sternum of adults. These wires can remain in the body as the sternum of an adult does not grow anymore. A preferable material for the wires is a high grade surgical steel alloy, for example steel in accordance with German industrial standard DIN 1.4441, which is an alloy chiefly consisting of iron, chromium, nickel and molybdenum. However, non-metallic material, such as synthetic material, can also be used as the surgical suture material. In the description of the present invention, the terms surgical suture and wire are used above and below synonymously and these can comprise metallic or non-metallic and absorbable or non-absorbable material, unless specifically stated otherwise.

For adolescent patients applying non-absorbable sutures is disadvantageous. As the sternum changes its size the sutures have to be removed to allow for the growth of the sternum. Therefore, a second operation has to take place which can be avoided by using absorbable material for the first surgical suture. As the bones of young patients generally heal more quickly and better, an additional reinforcement is not important.

For adults, it is recommended that six to eight wires are used, each having a length of 450 mm and a diameter of about 0,75 mm to 0,85 mm. The needles connected to the first surgical suture are preferably formed into a half-circle with a diameter of about 49 mm. The tip of the needles, i.e. a free end of the needle, is provided usually with a cutting edge.

Preferably, at least one length of second surgical suture is provided and connected to the first surgical suture in the vicinity of the ends of the first surgical suture. This second surgical suture is in general a surgical thread or threadlike material. The second surgical suture may be made of absorbable or non-absorbable material. As the second surgical suture is normally not intended to provide the primary force for the closure of the sternum, it is preferable that the second surgical suture is made of absorbable material. If it is made of felt-like material by itself, the second surgical suture may tampon the oozing haemorrhage by itself. Otherwise it serves as holder and a fixture for a first and/or second puncture closure and/or reinforcement, the latter being described in more detail further below. Then these puncture closure(s) overtake the tamponage and/or act as a reinforcement for preventing the first surgical suture from cutting into the bone when the first surgical suture is tightened. Of course it is not required, in the case the second suture is used as tamponage by itself, that a perfect seal is achieved. It is sufficient if a part of the oozing haemorrhage is retained and therefore the loss of blood is at least substantially reduced.

Different embodiments with respect to the number and shape of the second surgical suture are possible. In the following, various preferred embodiments are described in detail without limiting the invention to these embodiments. In particular, in all of these embodiments, the second surgical suture can be used, if desired, for the fixture of a puncture closure and/or reinforcement material and the application thereof at the sternum, or it may represent a puncture closure and/or reinforcement itself. Further, at least one connector connecting the ends of the second surgical suture to the first surgical suture in the vicinity of at least one end of the first surgical suture can be provided.

In a first preferred embodiment, one length of second surgical suture is provided. One end of the length of second surgical suture is connected to the first surgical suture in the vicinity of a first end of the first surgical suture and the second end of the second surgical suture is connected to the first surgical suture in the vicinity of the other end of the first surgical suture.

In a second preferred embodiment, two lengths of second surgical suture are provided. The first length is connected with one of its ends to the one end of first surgical suture in the vicinity of one end of the first surgical suture and the second length of the second surgical suture is connected with one of its ends to the first surgical suture in the vicinity of the other end of the first surgical suture. This second embodiment provides the advantage of better preventing the formation of undesirable loops of the second surgical suture on the posterior side of the sternum.

In a third preferred embodiment, two lengths of second surgical suture are provided. Each length of second surgical suture is formed into a loop and both ends of one length of second surgical suture are connected to the one end of the first surgical suture and both ends of the second length of surgical suture are connected to the other end of first surgical suture. Similar to the second embodiment, this third embodiment provides the advantage that undesired loops of the second surgical suture are unlikely to form on the posterior side of the sternum and, therefore, the sternal wire is easily applicable. Further, this third embodiment allows for providing additional puncture closures or seals on the anterior side of the sternum. These additional seals may be applied and fixed while connecting the free ends of the second surgical sutures.

All of the described embodiments may be provided with at least one first puncture closure and/or reinforcement for the posterior side of puncture channels. Usually, two first puncture closures are provided, one sealing for the opening of the one puncture channel and the other sealing for the opening of the other puncture channel on the posterior side.

Sealing in this context does not necessarily mean a perfect seal against blood loss. It forms part of the present invention that a blocking of the oozing haemorrhage is achieved with a puncture closure to the extent that a substantial part of the blood which may otherwise ooze on account of the wound at the puncture channel is prevented from doing so. The first puncture closure or a reinforcement may also reinforce the posterior outlet of the puncture channel thus preventing the first surgical suture from cutting into the cortical layer of the sternum. The puncture closures and/or reinforcements may be connected to the first and/or second surgical suture.

As revealed above, the first puncture closures and/or reinforcements may be connected only to the first surgical suture. In this case, a connecting element, for example based on friction, can be provided and connected to the first surgical suture for holding and correctly positioning the first puncture closures and/or reinforcements at the sternal plates. However, connecting the first puncture closure and/or reinforcement to the first surgical suture only may sometimes result in an inclination of the puncture closure and/or reinforcement after application thereof to the dorsal side of the sternum. Therefore, the haemostatic and/or reinforcing effect may not be optimal. Further, the application of the puncture closures and/or reinforcement only connected to the first surgical sutures is a rather difficult task as the surgeon has to ensure the correct position of the first surgical suture on the one hand and to position the puncture closures and/or reinforcements on the posterior side of the sternum on the other hand.

To overcome this problem, the first puncture closures and/or reinforcements are connected (additionally or only) to the second surgical suture(s). Principally, they may be connected to the first and second surgical suture(s) or to the second surgical suture(s), only. As the second surgical suture is usually a surgical thread, the first puncture closures and/or reinforcements can be threaded onto the second surgical suture(s). In the case that two second sutures are provided such that they are formed into loops and connected with both ends to the first surgical suture (third embodiment) no further holding means for holding the puncture closures and/or reinforcements in position is required. On the other hand, for the first and second embodiments, a holding means for holding the first puncture closures and/or reinforcements is required, e.g. knots or the like, to prevent the first puncture closures and/or reinforcements from falling off the suture and, upon application, to permit positioning of the puncture closures and/or reinforcements over the puncture channels.

In a preferred embodiment, the first puncture closures and/or reinforcements intended for sealing the dorsal openings of the puncture channels and/or reinforcing are connected to the second surgical suture only. This renders their application easy, especially in the cases of the second and third embodiment described above. After application of the first surgical sutures, the second surgical sutures can be tightened and the first puncture closures and/or reinforcements can be positioned without requiring previous or simultaneous correct positioning of the first surgical sutures.

The first puncture closures and/or reinforcements which are used for sealing the puncture channels on the dorsal side of the sternum may be made of absorbable or non-absorbable material. If it is not intended to reinforce the closure of the sternum but only to ensure a minimisation of the loss of blood by tamponing the oozing haemorrhage, an absorbable material which is absorbed after a certain time and, therefore, not longer available in the body is sufficient. For example, a felt-like material may be used. An example for an absorbable felt is a polyglycolic acid felt and an example for a non-absorbable felt is a teflon felt. The shape of the puncture closure and/or reinforcement can take any suitable form, such as a substantially planar rectangle, square or other polyon or can be circular or oval. Further, the puncture closure may have a shape which is not planar, i.e. be in the form of a tube, cone or the like.

However, in another alternative embodiment, by forming the second surgical suture in an appropriate manner, the first puncture closures may not be required, even though it is preferable to use them. In case no first puncture closures are provided, the second surgical suture is preferably of an absorbable felt-like material, e.g. a polyglycolic acid felt. This felt-like material can be shaped in order to ensure that a portion of the second surgical suture which, after its application, has a larger cross section at or near the posterior side of the sternum than a portion protruding from the anterior side of the sternum after application. This larger portion is able to seal the posterior opening of the puncture channel. To minimise the loss of blood due to an oozing haemorrhage without providing additional puncture closures, the second surgical suture may, for example have a conical shape such that the portion of the second surgical suture near the needle is of a smaller cross section than the portion located near the middle of the second surgical suture. After application of the first and second surgical suture to the sternum by advancing the first needle with the first ends of the surgical sutures through one sternal half and the second needle with the second ends of the second surgical sutures through the other sternal half and tightening the surgical sutures, the second surgical suture works as a stopper, being at least partially inserted in the puncture channels. Therefore, the oozing haemorrhage in the channels of the sternum becomes tamponed on the posterior side of the sternum as it cannot flow out through the openings of the puncture channels. It should be ensured that the largest cross-section of the second surgical suture(s) is big enough not to avoid being pulled through the puncture channels in the cortical layer of the sternum. A conical shape of the second surgical suture(s) is not absolutely required. Any shape varying in cross section or diameter is possible. The smaller cross section or diameter portions of the second surgical suture(s) render the application of the suture(s) easy in terms of pulling them through the puncture channels until the larger cross section or diameter portions intended for the sealing function come to rest in the puncture channel.

As it is necessary to separate the needles from the first and second surgical sutures and to separate the first surgical suture from the second surgical suture after passing the needles provided with the sutures through the sternum, the needles can be separated from the sutures by cutting the latter. The second surgical suture can be connected directly or by means of a connector to the needle and/or the first surgical suture in the vicinity of at least one end of the first surgical suture. This connection may be in a portion of the first surgical suture near the connection of the first surgical suture to the needle or, preferably, directly at the needles. If the at least one length of second surgical suture is connected directly with at least one needle, the second surgical suture(s) is/are separated automatically from the first surgical suture and the needles by separating the first surgical suture from the needles.

Preferably, the cross-sectional dimensions of the first and second surgical suture are different. The first surgical suture can have a first diameter D1 and the second surgical suture can have a second diameter D2 whereby D1 > D2. This is especially useful if puncture closures and/or reinforcements are provided. After connection of the loose ends of the first surgical sutures to close the sternum, it is still possible to pull the second surgical sutures, preferably made of thread material, through the puncture channels and to connect the pulled ends together to securely apply the puncture closures to the posterior side of the sternum. If the diameter D2 is too large, this may result in fouling and a correct application of the puncture closures is no longer possible. If the puncture closure has also a reinforcing function, the second surgical suture has to be pulled first in order to suitably position the puncture closure at the opening of the puncture channels such that the reinforcing function can be realized. As stated above, the second surgical suture is of absorbable material. After a certain time the second surgical suture may be absorbed as it is normally needed only to block the oozing haemorrhage. This means that all parts may be made of absorbable material, except for the needles.

Preferably, the connectors for connecting the first and second surgical sutures are provided with at least one opening adapted to accommodate at least one end of the first and/or second surgical suture. The first and second surgical sutures are preferably inserted into the openings so that one of the first and second surgical sutures extends further into the opening than the other. For this purpose one opening for both sutures or two separate openings may be provided. In these openings, one end of the first surgical suture and/or both ends of the second surgical suture may be inserted. Preferably, these openings are disposed at the end of the needles which are opposite to the tips of the needles. With the different extension of the sutures in the openings, it is possible to press a first portion covering the insertion with a pressure P1 and a second portion adjacent to this first portion with a pressure P2, whereby P2 > P1. The pressing portions should be arranged in such a manner that one of the portions covers the first and the second surgical suture and the other portion covers only one of the first and second surgical sutures. By applying different pressures, it is possible to avoid a squeezing or cracking of the second surgical suture. For fixing the first surgical suture having a diameter D1 > D2 and being formed of, for example, wire material, a higher pressure is needed than for fixing the second surgical suture, e.g. thread-like material. This different pressure can be applied in this embodiment.

Alternatively, it is possible that the sutures extend with an identical length into the openings and to ensure correct fixture only by pressing two different portions of the connector with two different pressures. Using openings which are well fitted to accommodate in a first portion both sutures and in a second portion only the first surgical suture, even a fixture with only one pre-determined pressure is possible.

Another type of connector comprises a sleeve covering one end of the first surgical suture and at least one end of the second surgical suture. After insertion of both sutures into the sleeve, the sleeve is connected to the needle. This connector is very versatile as deviations from a certain diameter of the surgical sutures can be tolerated.

For a better connection between the connector and the first and/or second surgical suture, adhesive material can be employed. This helps to avoid an undesired loss of any of the parts of the sternal cerclage during application.

The same advantages may be achieved using another embodiment of the present invetion according claim 8. In this case a sternal closure comprises only one single needle connected to a first surgical suture. Further, a second surgical suture is connected to the first surgical suture in the vicinity of the needle, such that it can be advanced simoultaneously together with the first surgical suture through a sternal half in the direction from posterior to anterior. This provides the advantage that both the first and second surgical sutures are positioned in one step and in the same puncture channel. Therefore, injury and blood loss is minimized. A puncture closure and/or a reinforcing element may be provided and connected to the second surgical suture for sealing the posterior opening of a puncture channel. For this purpose, the second surgical suture may be connected with both of its ends to the first surgical suture or only with one of its ends. In this aspect of the present invention, two sternal closures are required at each position to form a cerclage if the direction of advancing the needle is from the posterior to the anterior side. The posterior ends of the two sternal closures are then connected during surgical treatment before or after application thereof to the sternum. After application and connection at the posterior side of the sternum the sternal closures are tightened and connected on the anterior side of the sternum.

As for the closure of a sternum of an adult, six to eight sternal cerclages are usually necessary. For this purpose, a sternal closure kit may be provided comprising a plurality of sternal closures, for example eight sternal closures. As already mentioned, it may be desirable to additionally tampon the anterior openings of the puncture channels and/or to reinforce the sternal closure on the anterior side. Therefore, supplementary second puncture closures may be provided separately from the sternal cerclages with the sternal closure kit. These may be applied to the anterior side of the openings of the puncture channels when connecting the ends of the second surgical sutures. Similarly, additional reinforcements may be provided separately and may be applied to the anterior side of the openings of the puncture channels when connecting the ends of the second surgical sutures. If formed appropiately, the additional puncture closures and/or reinforcements may be also appllied to the posterior side of the openings of the puncture channels.

For all described embodiments with first and second surgical sutures, the mode of application is similar. Firstly, the first surgical suture and the second surgical suture(s) are advanced from the posterior side through the sternum using the needles until they protrude from the cortical layer on the anterior side. The one needle with the one end of the first surgical suture and at least one end of the second surgical suture is advanced through one sternal half and the other needle with the other end of the first surgical suture with at least one end of second surgical suture is advanced through the other sternal half. Subsequently, the needles are separated from the surgical sutures and the first or second surgical suture is tightened. In this procedural step, the correct seat of the first surgical suture on the dorsal side can be checked, e.g. by holding the ends of the first surgical suture and moving these to and fro. Following this, the ends of the first surgical suture are tightened and twisted together.

If second surgical sutures are provided, these can be tightened first or afterwards, thereby applying the first puncture closures and/or reinforcements to the openings of the punctures channels on the posterior side of the sternum. These puncture closures and/or reinforcements may be a separate element connected to the second surgical suture(s) or be integrated in the second surgical suture(s) as outlined above.

The ends of the second surgical sutures are connected first or afterwards. Depending on the embodiment, it is possible to connect the one end of one length of surgical suture to the other end of the same length of surgical suture or to connect the ends of second surgical sutures, for example, crosswise.

In this point, the third embodiment is specially advantageous. Connecting the ends of the second surgical sutures, it is possible to easily provide second puncture closures and/or reinforcements on the anterior side of the sternum and to seal and/or reinforce the openings of the puncture channels on the anterior side. This is, however, not necessary in every case. If a sufficient amount of muscular tissue is present on the anterior side of the opening of the puncture channels (big pectoralis major muscle or muscle insertion of the pectoralis major muscle), a second puncture closure is not necessary or even disadvantageous, when using only a haemostatic puncture closure. Including a small felt-like plate, e.g. a polyglycolic acid plate, may result in a compression of muscular tissue and would lead to a necrosis. In this case, the muscular tissue overtakes the sealing function of the ventral openings of the puncture channels. In case of puncture closure with a reinforcing element or a reinforcement only, the surgeon has to judge possible advantages against possible disadvantages.

The first and second puncture closures may have different shapes. In a simple embodiment such a clousure is made of only one element. For this element preferably an haemostatic material, e.g. an absorbent felt-like material, is employed. The puncture closure should be large enough to securely close the opening of the puncture channel. Its shape may be circular, rectangular, irregular and so on. The first and second elements may be connected to each other by means of an adhesive material, a form closure or the like or they may be provided separately.

Preferably, the puncture closure comprise a reinforcement. This reinforcement, which constitutes a second element of the puncture closure, has a higher rigidity than the first element. The second element may be made of absorbable or non-absorbable material and, therefore, the entire puncture closure may be absorbable or non-absorbable. Further, the reinforcing element may have a certain flexibility. This flexibility improves the adaptation to the surface of the sternal half upon which it is placed and, thereby, the load distribution and application thereof. However, a flexibility of the reinforcing element is not absolutely required so that it can also be rigid. The presence or absence of flexibility, for example, depends on the shape of the puncture closure. The second element may have a similar shape as the first element and the first and second element are preferably connected to each other and, more preferably, integrally formed. In the case they have similar shapes, the second element may cover one surface of the first element. However, it also possible that the second element or the first element cover only parts of the other element or have completely different shapes.

A reinforcement of the puncture closure helps to provide a good distribution of the force or load exerted by the tightened first surgical suture onto the sternum near the puncture channel. This minimizes the danger that the surgical sutures cut into the cortical layers and, therefore, the formation of a larger puncture or incision. Such may lead to deep wound infection and an instable osteosynthesis. A very effective prevention of an incision may be achieved if the first and/or second surgical sutures are passed through the reinforced puncture closure. Then the reinforced element distributes the acting forces to a bigger portion of the sternum and, therefore, the danger of an incision becomes minimized.

Preferably, the puncture closure comprise a second element having a larger surface area than the first element. The puncture closure is applied to the sternum in such a manner that the first element of the puncture closure contacts the sternum and is of a sufficient size to serve as a tamponage for the oozing haemorrhage. The larger second element, i.e. the reinforcing element, is positioned with respect to the sternal half such as to provide for good load distribution. Of course, the first haemostatic element may be larger than the second reinforcing element as long as the second element ensures a good load distribution.

In another preferred embodiment, providing protrusions on the puncture closure may improve the reinforcing effect of the puncture closure and, therefore, the reinforcing effect of the entire surgical suture system. The puncture closure comprising protrusions may be applied in such a manner that the protrusions incise into a sternal half in the vicinity of a puncture channels or that a protrusion extends into a puncture channel. In these cases, the reinforcing effect is very good as a distribution of the load is not only achieved on the surface of the sternal halves but also in the direction of the depth of the halves so as to be able to absorb forces acting at right angles to the surface of the sternum. In case the protrusion is arranged to extend into a puncture channel, it is preferable that the reinforcing second element is formed and disposed in such a manner that it also extends into the puncture channel. The reinforcing second element in this case may be at least partly rod-shaped, in the form of a sleeve or the like.

Preferably, the reinforced puncture closure has an opening provided at least in the reinforcing second element but, preferably, also in the haemostatic first element, such that there is at least a partial overlap of the openings to form a through-hole. This opening renders the application of the puncture closure easier as the suture material need not be pushed through the puncture closure material. This is especially advantageous if a protrusion in the central portion of the puncture closure is provided through which the suture(s) have to be passed. This opening is preferably circular. In this case, the opening may be used in the same manner as a buttonhole. In another peferred embodiment, the opening is, for example, slit-like in shape and extends from the peripheral edge of the puncture closure towards the center. A slit-like opening is advantageous as such a puncture closure may be applied after applying the sutures to the sternum but before tightening them. Alternatively, if the puncture closure is threaded onto the first and/or second surgical suture, the opening can be located at a distance from the peripheral edge of the puncture closure such that it is completely surrounded thereby.

Substantially, the aforesaid properties of a puncture closure may be adapted to a element having a reinforcing function only, too.

Of course, the invention may be used together with an alternatively reinforcing sternal closure system comprising staples applicated by a staple gun or the like. If second puncture closures for closing the puncture channels and/or reinforcements are present on the anterior side of the sternum, the staples reinforcing at the anterior side of the sternum may be applied to the sternum before or after fixing the second puncture closures and/or reinforcements, or the puncture closures and/or reinforcements and the staples may be fixed simultaneously. The latter is preferable, as the puncture closures and/or reinforcements may be fixed exclusively by pushing the staples through the second puncture closures and/or reinforcements or in combination with a fixture by the second surgical sutures.

### Brief Description of the Drawings

In the following, the present invention is described by way of exemplary embodiments referring to the accompanying figures, in which:
Fig. 1 shows a cross-sectional view of a closed sternum following mid-line thoracotomy using a sternal cerclage according to a first embodiment of the present invention or the prior art;
Fig. 2 shows a cross-sectional view of a closed sternum following mid-line thoracotomy using a sternal cerclage according to another embodiment of the present invention;
Fig. 3 shows a cross-sectional view of a closed sternum following mid-line thoracotomy using a sternal cerclage according to a further embodiment of the present invention;
Fig. 4 shows a perspective view of a closed sternum following mid-line thoracotomy using sternal cerclages according to the present invention seen from the posterior side;
Fig. 5 shows perspective view of a closed sternum following mid-line thoracotomy seen from the anterior side using sternal cerclages according to different embodiments of the present invention;
Fig. 6a shows a first preferred embodiment of a sternal wire according to the present invention;
Fig. 6b shows a second preferred embodiment of a sternal wire according to the present invention;
Fig. 6c shows a third preferred embodiment of a sternal wire according to the present invention;
Fig. 6d shows a fourth preferred embodiment of a sternal wire according to the present invention;
Fig. 6e shows a fifth preferred embodiment of a sternal wire according to the present invention;
Fig. 7 shows an enlarged view of a needle connected with the surgical sutures of an embodiment of sternal wire according to the present invention;
Fig. 8 shows an embodiment of a sternal closure kit according to the present invention;
Fig. 9 shows an exemplary preferred embodiment of a puncture closure according to the present invention;
Fig. 10 shows a second preferred embodiment of a puncture closure according to the present invention;
Fig. 11 shows a cross-sectional view of a puncture closure according a third preferred embodiment of the present invention;
Fig. 12 shows a cross-sectional view of a puncture closure according a fourth preferred embodiment of the present invention; and
Fig. 13 shows a cross-sectional view of a puncture closure according a fifth preferred embodiment of the present invention.

### Detailed description of preferred exemplary embodiments of the present invention

Fig. 1 shows a sternum consisting of two sternal halves 10a, 10b. Following a mid-line thoracotomy, the two sternal halves 10a, 10b have been separated. In each sternal half, a puncture channel 11a, 11b resulting from advancing a needle through the cortical layers of the sternum can be seen. The sternal halves are tightened together by a surgical wire forming a cerclage. This wire extends from the mid-line of the sternum on the anterior side 13 through the first puncture channel 11a to the posterior side 15 of the sternum and from there through the second puncture channel 11b back to the anterior side where the two ends of the surgical wire 12 are connected. It can be seen from Fig. 1 that the openings of the puncture channels 11a, 11b on the posterior side 15 of the sternum are not covered or tamponed.

Disposing a needle at each of the two ends of the wire 12 ensures an easy application of the wire 12 to the sternum. It is not neccessary to grasp the needle on the posterior side of the sternum as the wire 12 is advanced through both sternal halves in the direction from the dorsum to the ventrum. Grasping the needle at the posterior side of the sternum may be difficult, as the posterior side of the sternum is not freely accessible and a plurality of wires 12 normally has to be applied for closing an opened sternum. Further, the danger is reduced that a dull and/or bent needle becomes jammed during application of the suture system at the second sternal half. This may result in an enlarged puncture channel and/or in a teared edge of the puncture channel when having to apply excessive force to further advance the needle or remove the needle from the sternum. This in turn results in an additional blood loss. Providing a needle at both ends of the first surgical suture minimizes this risk and, therfore, helps to ensure a minimization of blood loss.

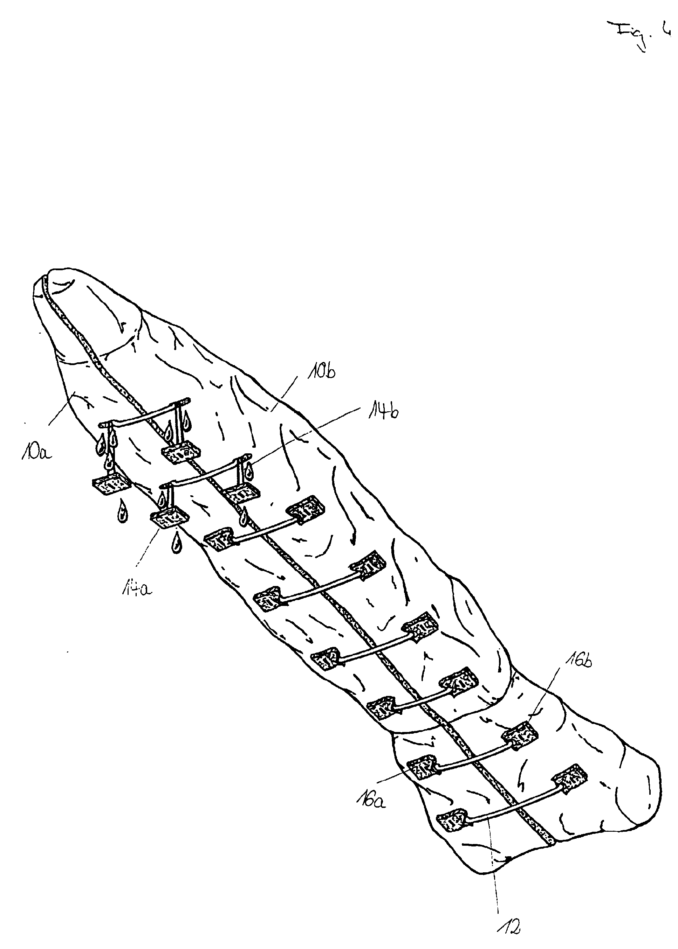

Fig. 2 shows the same sternal cerclage as Fig. 1. Additionally, in each of the puncture channels 11a, 11b, a second surgical suture 14a, 14b, in particular a thread, is inserted. This thread extends from the posterior to the anterior side of the sternum. A first puncture closure 16a, 16b fixed to the threads 14a, 14b covers the openings of the puncture channels 11a, 11b on the posterior side 15 of the sternum. The opposite opening of the puncture channels, i.e. the openings on the anterior side 13 of the sternum, are also covered by second puncture closures 18a, 18b. These second puncture closures 18a, 18b are fixed by tightening and connecting the ends of the second sutures 14a, 14b protruding on the anterior side of the sternum. The first length of the second suture 14a forms a loop inside the first puncture channel 11a which is provided at the opening on the posterior side 15 and the opening on the anterior side 13 with one puncture closure 16a, 18a, respectively. The second length of surgical suture 14b is disposed in the same manner in the second puncture channel 11b.

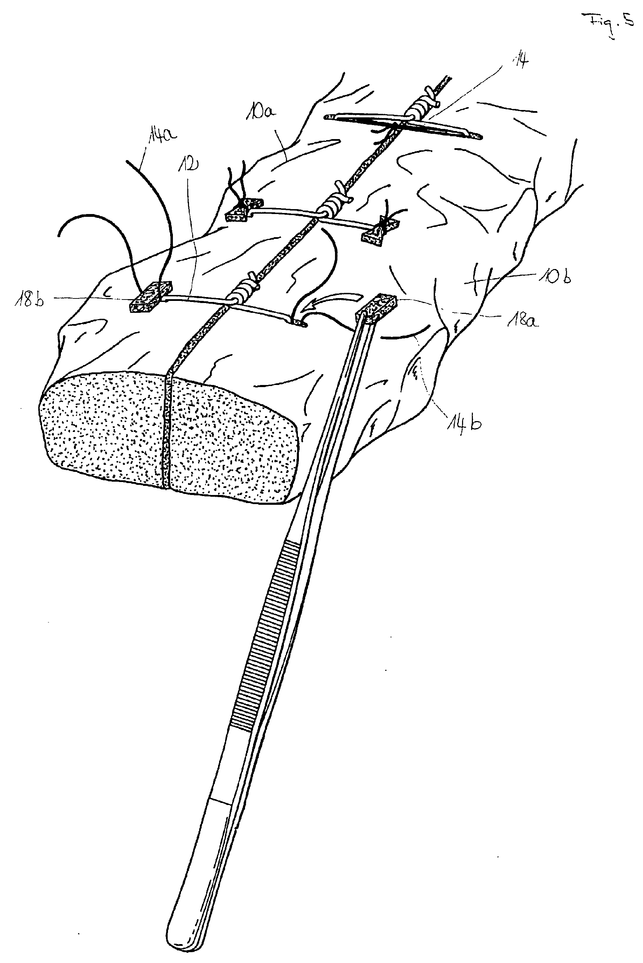

Fig. 3 shows a cross-section of a sternum similar to Fig. 1. Additionally, two lengths of second surgical sutures 14a, 14b are provided. A puncture closure 16a, 16b is disposed on each of the lengths of second surgical suture 14a, 14b. The second surgical suture together with the puncture closure 16a, 16b is disposed at the posterior side of the sternum, so that the puncture closures cover the openings of the puncture channels 11a, 11b on the posterior side of the sternum and the sutures extend from there through the puncture channels to the anterior side of the sternum, whereby a first length of second surgical suture 14a is connected to a second length of second surgical suture 14b on the anterior side of the sternal plates.

Additionally, reinforcements (not shown) can be provided on the posterior and/or anterior side of the sternal plates, which is advantageous, if the puncture closures 16, 18 (shown in Fig. 2 and 3) are not reinforcing puncture closures.

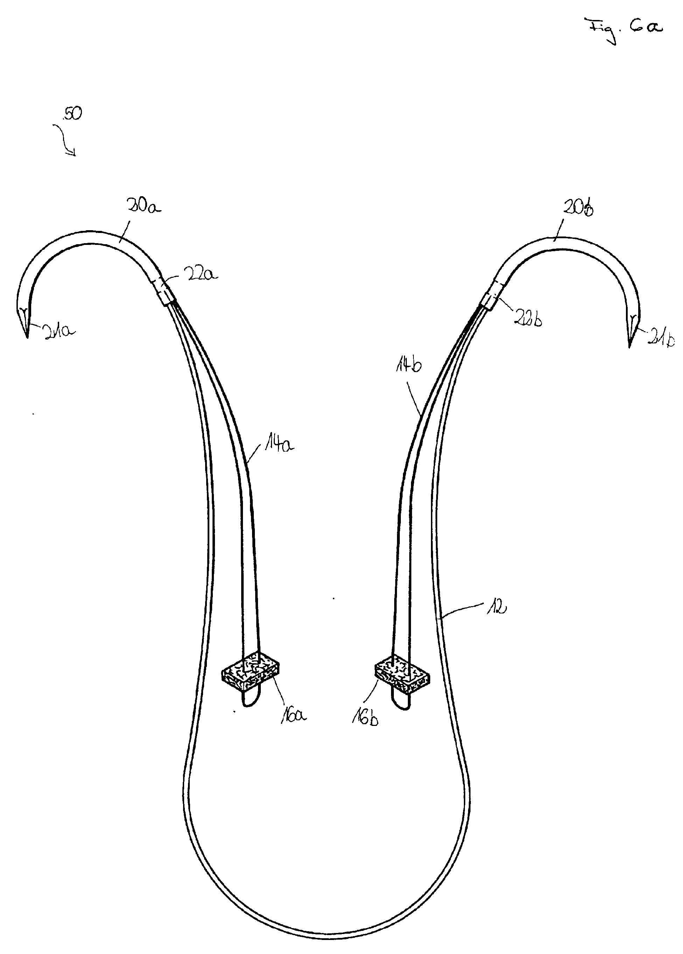

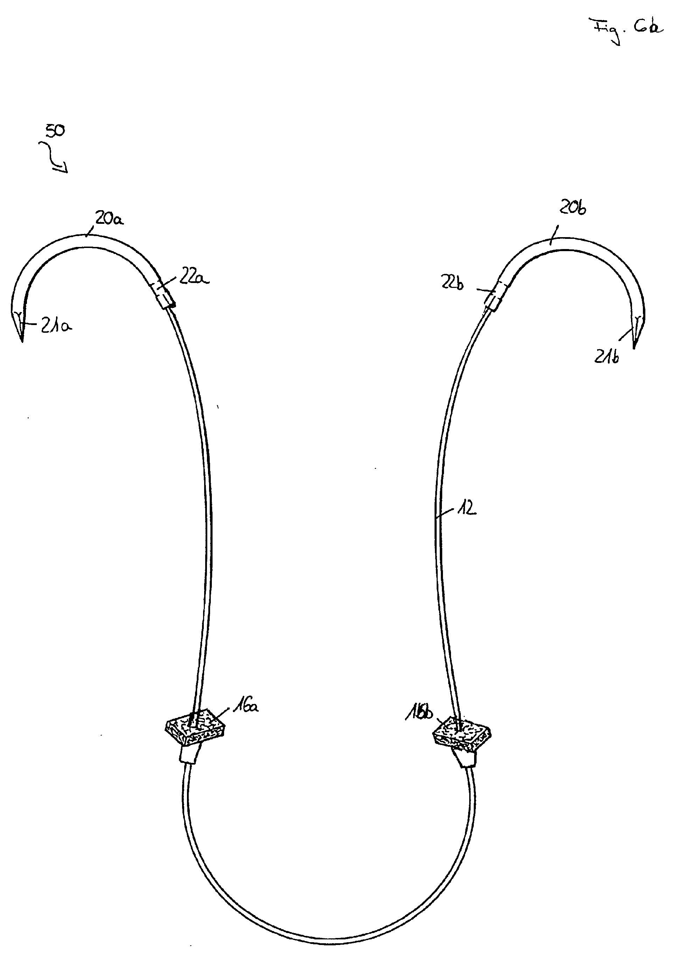

Fig. 4 shows a complete sternum seen from the posterior side. Eight sternal cerclages are applied for closing the sternum following mid-line thoracotomy. Each sternal half 10a, 10b is provided with eight puncture channels in which wires 12 are inserted. The openings of the puncture channels on the posterior side are closed by first puncture closures, formed as rectangular felt-like pieces 16a, 16b. At two sternal cerclages, these puncture closures are shown in an incompletely fixed position. With the help of the two second sutures 14a, 14b, they have to be fastened and applied to the posterior side of the sternal plates. Of course it is possible, as shown in Fig. 5, that not all openings of the puncture channels on the posterior and/or anterior side of the sternum are closed by puncture closures and/or reinforcements. This is especially helpful on the anterior side, where the pectoralis major muscle may overtake a part of the sealing function. In this case the surgeon has to judge advanteges and possible disadvantages (necrosis of muscular tissue) before application of the puncture closures and/or reinforcements.

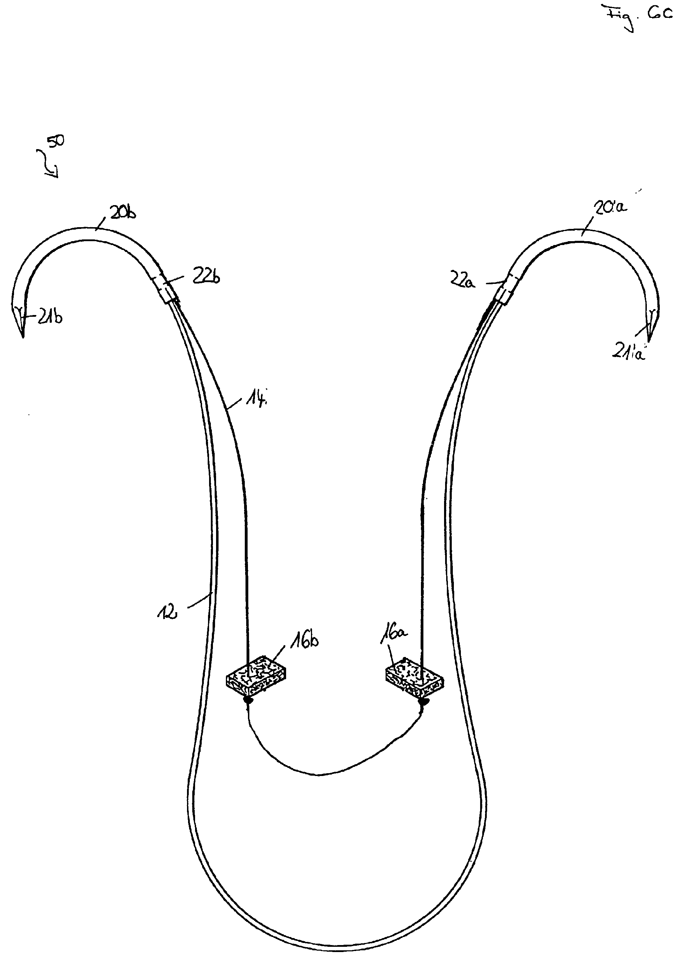

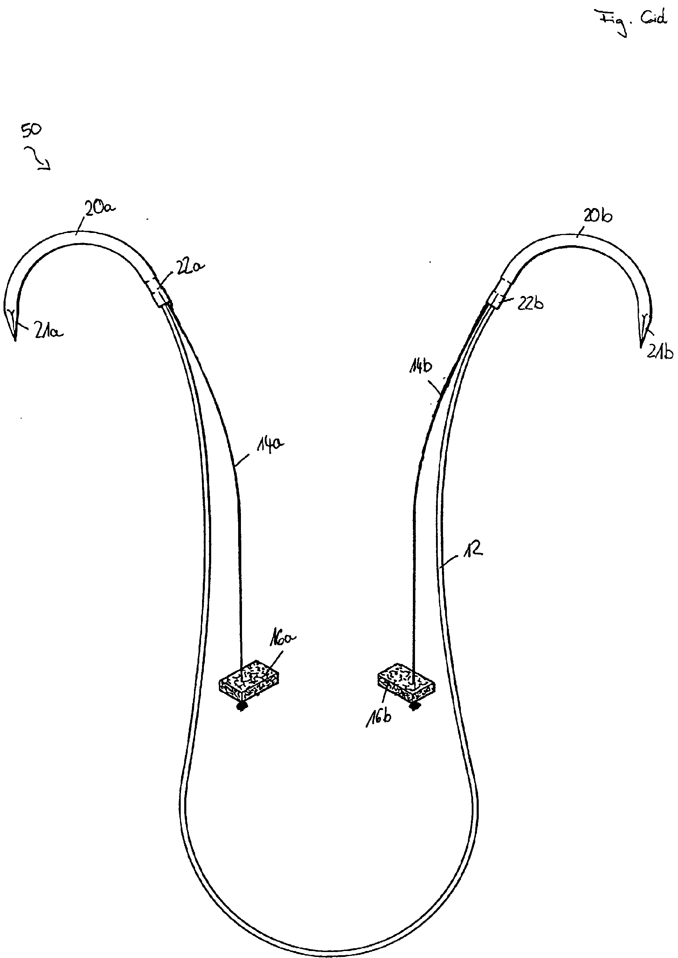

Figs. 6a to 6e and 7 show a first surgical suture 12 according to the present invention in particularly preferred embodiments. In the embodiments according Figs. 6a to 6d, the first surgical suture 12, for example, is made of wire material and has a length of about 450 mm and a diameter D1 of about 0,75 mm to 0,85 mm. For the embodiment according Fig. 6d, for example, a length of about 250 mm of wire material is required. Other suitable lengths and diameters may of the first surgical suture may be used, too. In Figs. 6a to 6d, two connectors 22a, 22b are disposed at the ends of the first surgical suture 12 and connect the first surgical suture 12 to two needles 20a, 20b. In the shown preferred exemplary embodiment, the needles 20a, 20b are bent and form a half circle having a diameter of about 49 mm. A larger diameter or a substantially straight form of the needles is also possible, however, not particularly advantageous. The tips of the needles 21a, 21b are provided with cutting edges. According Fig. 6a, the connectors 22a, 22b further accommodate two ends of two lengths of second surgical suture 14a, 14b, thereby the first connector 22a accommodates both ends of a first length of second surgical suture 14a and the second connector 22b accommodates both ends of a second length of second surgical suture 14b. The second surgical suture, for example, a surgical thread, has a diameter D2 which is smaller than the diameter D1 of the first surgical suture 12. Two first puncture closures 16a, 16b are provided. The first puncture closure 16a is disposed on the first length of the second surgical suture 14a and the second puncture closure 16b on the second lengths of surgical suture 14b. A second surgical suture 14a, 14b is led through each of the puncture closures 16a, 16b two times so as to form a loop and to fix the puncture closure 16a, 16b to the second surgical sutures 14a, 14b.
Other preferred embodiments are not illustrated by an accompagnying figure. However, the lengths of second surgical suture 14a, 14b may, for example, be fixed only at one of their ends to the first surgical suture 12 (Fig. 6d) or only one length of second surgical suture may be provided, each end thereof beeing fixed to the first surgical suture 12 (Fig. 6c). In this case, preferably one end of the second surgical suture 14 is fixed to the first surgical suture 12 in the vicinity of the first needle 20a and the other end of second surgical suture 14 is fixed to the first surgical suture 12 in the vicinity of the second needle 20b. An embodiment without the use of a second surgical suture is shown in Fig. 6b.

As shown in Fig. 6e, it is also possible to provide only one needle 20 disposed at one end of the first surgical suture 12. The second surgical suture 14 may be connected to the first surgical suture with both of its ends as shown in Fig. 6e or only with one of its ends, similar to the embodiment shown in Fig. 6d.

In the exemplary embodiments, shown in Figs. 6a to 6e, the puncture closures are rectangularly shaped and, for example, are made of felt-like material such as a polyglycolic acid felt. Of course, another material, for example a teflon felt, may be used, too. The puncture closures 16a, 16b, the second surgical sutures 14a, 14b and the first surgical suture 12 can all be made of absorbable or non-absorbable material. Therefore, the whole sternal cerclage except the needles may be absorbable.

Fig. 7 shows the connector connecting the ends of the first and second surgical sutures to the needle 20. The connector 22 comprises one opening 24, which is adapted to accommodate one end of the first surgical suture 12 and both ends of the second surgical suture 14. Of course, more than one opening may also be provided. A pressing portion 28 is provided so as to secure the first surgical suture 12 and the second surgical suture 14 extending inside the pressure portion to the needle 20. Other types of connectors are possible. For example, rings or hooks can be formed accordingly or the sutures may be fixed by adhesive connection, only.

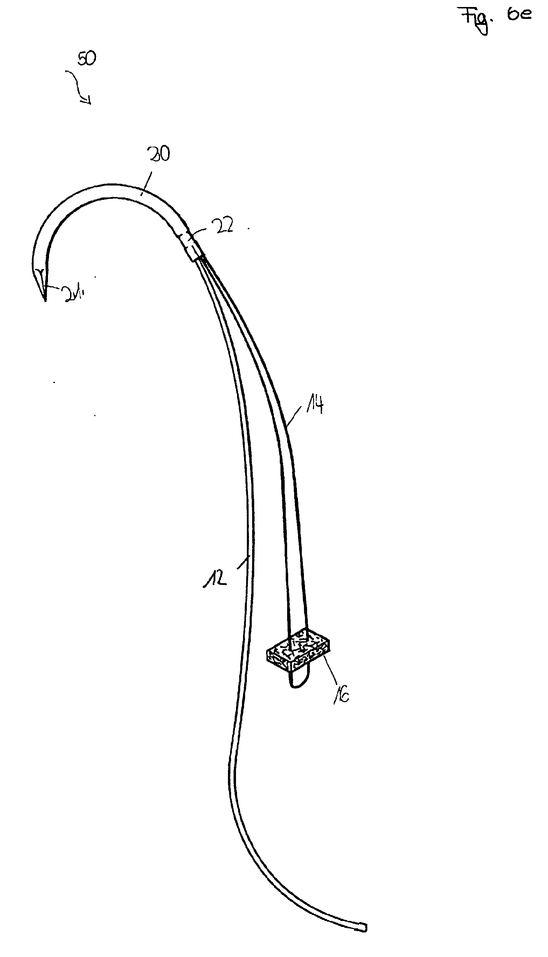

Fig. 8 shows an example of a sternal closure kit comprising a plurality of sternal wires 50 according to Fig. 6a and additional second puncture closures 18. Naturally, any type of sternal wire in accordance with the present invention may be provided in a kit. In general, all the parts of the sternal closure kit are sterilised, for example by gamma irradiation, and are contained in a sterile blister pack 32. By way of example, the sternal closure kit may comprise 6 or 8 wires. This number can vary according to the embodiment of the sternal closure. In the case the needles are disposed at one end only, twice the amount of wires may be required as compared to the embodiments with needles at both ends.

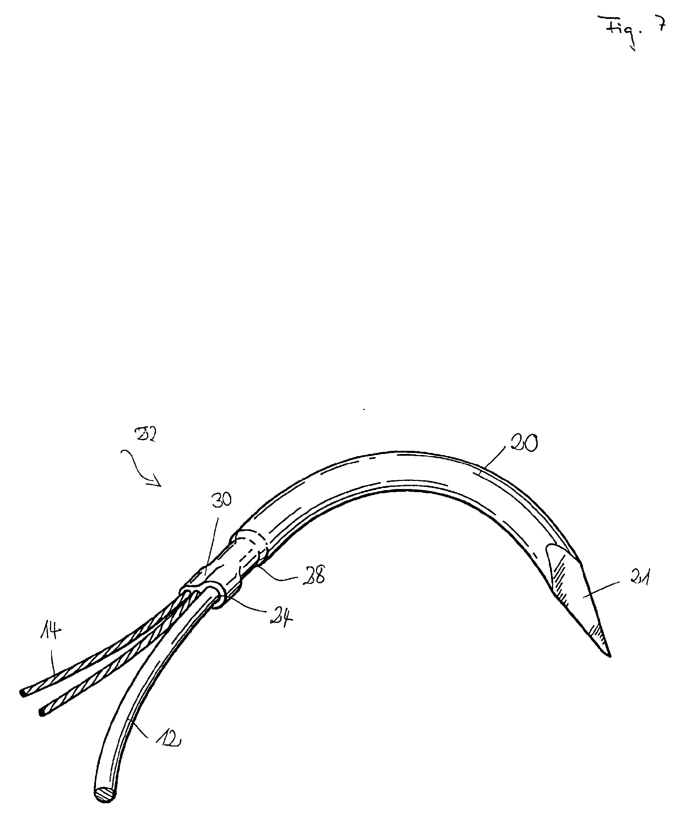

Fig. 9 to 13 show preferred exemplary embodiments of the first and second puncture closures 16,18. In principal each of the shown embodiments can be used as a first or a second puncture closure 16,18.

Fig. 9 shows a puncture closure having a substantially circular and planar geometry. A first element 60 comprises haemostatic material, which is possibly also absorbent and/or absorbable, and is connected to a second element 62. In this embodiment, the second element has the same shape as the first element. However, this is not absolutely required. The second element may only cover parts of the first element or be larger than the first element. The second element 62 is made of a reinforcing material, i.e. the material of the second element has a higher rigidity than the material of the first element 60. A circular opening 64 is provided through which a first and/or second surgical suture may be advanced. Naturally, the opening 64 can also have a different shape.

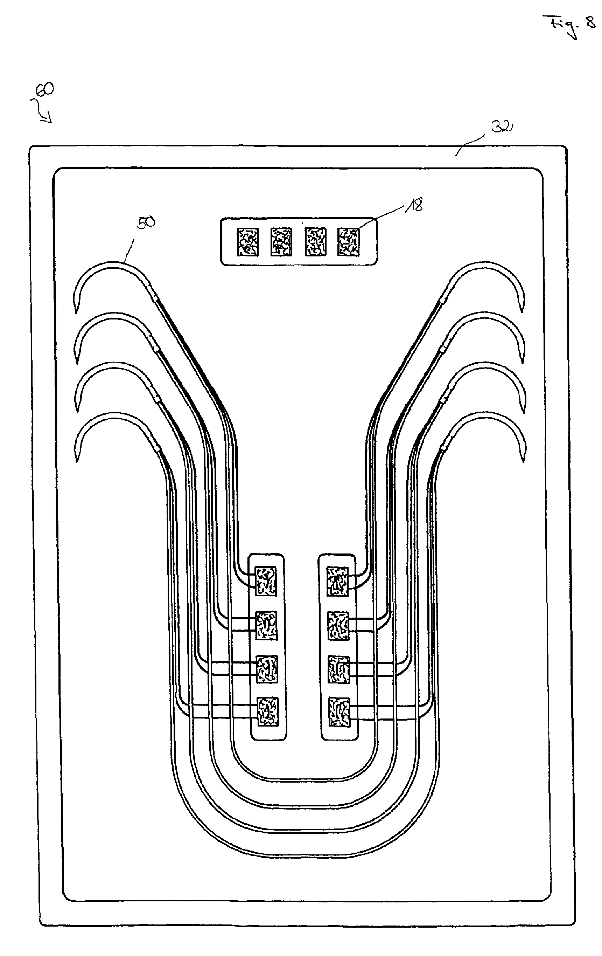

Fig. 10 shows a similar embodiment of a puncture closure as shown in Fig. 9. However, in this embodiment, the shape of the first and second elements 60,62 is rectangular. Further, a slit-like opening 66 is provided. This opening extends to the periphereal edge of the puncture closure which may be used for introducing a surgical suture for fixing the puncture closure. Other geometries of the puncture closure such as polygons, oval shapes and irregular shapes, are, of course, also possible.

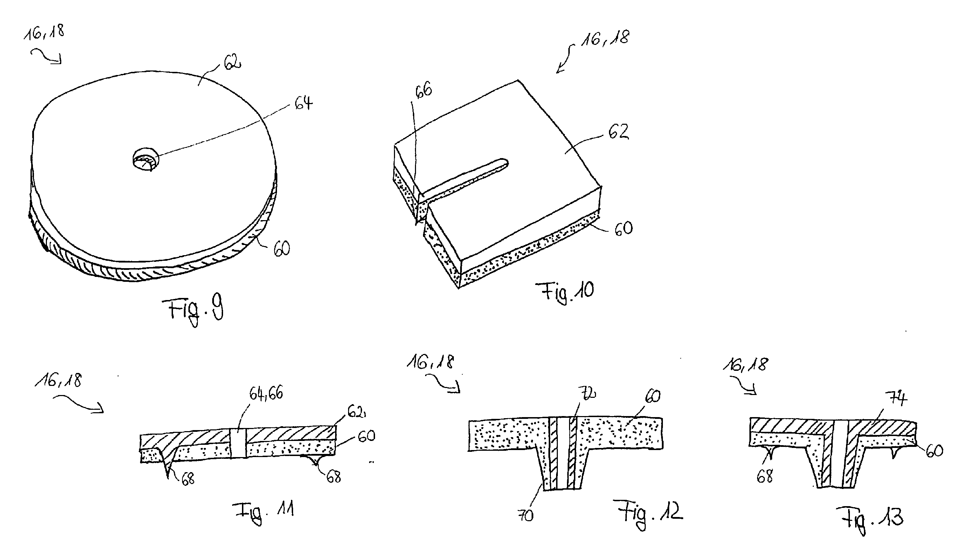

Fig. 11 to 13 show cross-sectional views of a further preferred embodiment of a puncture closure according to the present invention. In Fig. 11 a puncture closure is shown, in which a first element in the form of an haemostatic layer 60 is connected to a second element in the form of a reinforcing layer 62. An opening 64 for advancing a surgical suture, e.g. a wire, is provided. The opening 66 may be a circular opening or a slit-type opening as previously described with reference to Fig. 9 and 10, respectively, or of another shape. Additionally, protrusions 68 are provided. In this preferred embodiment, the protrusions are not located in the center portion of the puncture closure, as they are intended to slightly incise into the cortical layers of the sternum to securely fix the puncture closure in position and to further reinforce the sternal closure system by preventing movement of the puncture closure upon tightening of the suture(s). The protrusions may be made of the same material as the reinforcing layer or they may be made of different material and may possibly be provided with cutting edges.

Fig. 12 shows a cross-section of a further embodiment of a puncture closure comprising a planar haemostatic portion 60 formed so as to permit good coverage the puncture channel. The haemostatic portion 60 further comprises a protrusion 70 which is positioned in a center portion of the puncture closure and, after application, projects into the puncture channel. This produces a particularly advantageous effect of further enhancing the stilling of blood loss out of the puncture channel. A reinforcing sleeve 72 is disposed in the puncture closure so as to reinforce the protrusion and improve the distribution of the loads generated by tightening the surgical sutures in that it resists movement of the puncture closure because it sits securely in the puncture. The opening of the sleeve most suitably serves as the passage for the surgical sutures.

Fig. 13 shows a similar configuration to that of Fig. 12. Additionally the reinforcement 74 of the puncture closure extends over the entire surface of the puncture closure. Therefore, a better load distribution and reinforcement of the sternal closure can be achieved. Further, additional protrusions 68 as described with reference to Fig. 11 are provided. These protrusions may incise minimally into the cortical layers and, therefore, provide for a better hold of the puncture closure.
It should be noted that in all described embodiments, the portions of the puncture closure contacting the sternal halves can consist of haemostatic, for example absorbent, material. This is advantageous to hinder the oozing haemorrhage. On the contrary, the reinforcement of the sternum is achieved even if the reinforcing portions of the puncture closure do not directly contact the sternal halves.

Finally, it is also in accordance with the present invention that the puncture closure 16,18 only comprises a reinforcement the same as the second element of the puncture closure embodiments described above. Such a puncture closure 16,18 in the form of a reinforcement provides the same load distributing effects previously outlined and, although to lesser extent, also acts to at least some extent, as a barrier to blood loss if it is positioned at the puncture channel.

In the following, the application of an exemplary embodiment of the inventive haemostatic and/or reinforcing surgical suture system for sternal cerclages is described with reference to Fig. 5. In a first step, the sternal wires are passed through the sternal halves 10a, 10b. The first needle of one sternal wire 20a is passed through the first sternal half 10a in direction from posterior to anterior until portions of the first and second surgical sutures protrude on the anterior side of the sternal half 10a. The second end of the first and second surgical sutures is passed through the second sternal half 10b using the needle 20b in direction from posterior to anterior until the first and second surgical sutures protrude with a certain length from the second sternal half. In a second step the needles are separated from the surgical sutures. Then the first surgical suture is tightened. The surgeon checks before twisting the ends of each length of surgical suture if any undesired loops are formed on the dorsal side of the sternum by performing, e.g. a to and fro movement by alternatively pulling the two ends of the first surgical suture 12. Each length of first surgical suture 12 is fixed in this way to the sternum and, therefore, the sternum is closed. Then, using for example a sternal wire with the second surgical sutures according to Fig. 6a, the second surgical sutures 14a, 14b are tightened. Thereby, the first puncture closures 16a, 16b are pulled against the posterior side of the openings of the puncture channels and tampon the oozing haemorrhage. Before connecting the ends of the second surgical sutures 14a, 14b, a second puncture closure 18a, 18b may be applied on the ventral side of the openings of the puncture channels. As can be seen from Fig. 5, this sealing means on the anterior side of the sternal halves may be omitted. This is especially the case if the pectoralis major muscle has sufficient muscular tissue at the location of the puncture. Then this tissue overtakes the sealing function on the anterior side. In this case, the ends of the second surgical sutures 14a, 14b are directly connected together without adding second puncture closures 18a, 18b.

An advantageous aspect of the sternal cerclage according to the present invention lies in the fact that a seal can easily be provided to minimise the loss of blood due to a oozing haemorrhage inside the puncture channels. This task is achieved by providing a sternal wire with two needles disposed at the ends of a first surgical suture.
Additionally, first and/or second puncture closures may be provided and fastened to the sternal cerclage either separately or with the help of the first surgical suture or a separate second surgical suture.

## Claims

1. Haemostatic and/or reinforcing surgical suture system for sternal cerclages, comprising:
a length of first surgical suture having respective ends; and
a needle disposed at each end of said first surgical suture.

2. Haemostatic and/or reinforcing surgical suture system for sternal cerclages according to claim 1, further comprising:
at least one length of second surgical suture having respective ends, wherein
at least one end of the second surgical suture is connected to the first surgical suture in the vicinity of one end of said first surgical suture.

3. Haemostatic and/or reinforcing surgical suture system for sternal cerclages according to claim 1, further comprising:
at least one first closure connected to said first surgical suture.

4. Haemostatic and/or reinforcing surgical suture system for sternal cerclages according to claim 2, further comprising:
at least one first closure connected to said first and/or second surgical suture.

5. Haemostatic and/or reinforcing surgical suture system for sternal cerclages according to claim 4, characterized in that
two lengths of said second surgical suture having respective ends are provided;
said lengths of second surgical suture are formed into loops;
at least two first closures are provided, one connected to the one length of said second surgical suture and the other one connected to the other length of said second surgical suture; and
both ends of the one length of second surgical suture are connected to said first surgical suture in the vicinity of the one end of said first surgical suture and
both ends of the other length of second surgical suture are connected to said first surgical suture in the vicinity of the other end of said first surgical suture.

6. Haemostatic and/or reinforcing surgical suture system for sternal cerclages according to claim 4, characterized in that
one length of said second surgical suture having respective ends is provided;
said length of second surgical suture is formed into a loop;
at least two first closures are provided and connected to said length of second surgical suture; and
the one end of said length of second surgical suture is connected to said first surgical suture in the vicinity of the one end of said first surgical suture and
the other end of said length of second surgical suture is connected to said first surgical suture in the vicinity of the other end of said first surgical suture.

7. Haemostatic and/or reinforcing surgical suture system for sternal cerclages according to claim 4, characterized in that
two lengths of said second surgical suture having respective ends are provided;
at least two first closures are provided, one connected to the first length of said second surgical suture and the other one connected to the other length of said second surgical suture; and
one end of one of said lengths of second surgical suture is connected to said first surgical suture in the vicinity of the one end of said first surgical suture and one end of the other of said lengths of second surgical suture is connected to said first surgical suture in the vicinity of the other end of said first surgical suture.

8. Haemostatic and/or reinforcing surgical suture system for sternal cerclages, comprising:
a length of first surgical suture having respective ends;
a needle disposed at one end of said first surgical suture;
at least one length of second surgical suture having respective ends;
wherein at least one end of the second surgical suture is connected to the first surgical suture in the vicinity of the one end of said first surgical suture.

9. Haemostatic and/or reinforcing surgical suture system for sternal cerclages according any one of the proceding claims, characterized in that
said first surgical suture is made of absorbable or non-absorbable material.

10. Haemostatic and/or reinforcing surgical suture system for sternal cerclages according to claim 8 or 9, further comprising:
at least one first closure connected to said first and/or second surgical suture.

11. Haemostatic and/or reinforcing surgical suture system for sternal cerclages according to any one of the preceding claims 3 to 7 and 10, characterized in that
said at least one first closure is made of absorbable and/or non-absorbable material.

12. Haemostatic and/or reinforcing surgical suture system for sternal cerclages according to any one of the claims 3 to 7 and 11, characterized in that
said at least one first closure comprises a reinforcing member.

13. Haemostatic and/or reinforcing surgical suture system for sternal cerclages according to any one of the claims 2 to 12, characterized in that
said second surgical suture is made of absorbable or non-absorbable material.

14. Haemostatic and/or reinforcing surgical suture system for sternal cerclages according to anyone of the claims 2 to 13, characterized in that
said first surgical suture has a first diameter d1 and
said second surgical suture has a second diameter d2 different from the first diameter d1.

15. Haemostatic and/or reinforcing surgical suture system for sternal cerclages according to claim 14, characterized in that d1 > d2.

16. Sternal closure kit, comprising
a plurality of haemostatic and/or reinforcing surgical suture system for sternal cerclages according to anyone of claims 1 to 15.

17. Sternal closure kit according claim 16, further comprising
a plurality of second closures provided separately from said haemostatic and/or reinforcing surgical suture system for sternal cerclages.

18. Implantable closure for a haemostatic and/or reinforcing surgical suture system for sternal cerclages , comprising
a first element of haemostatic material mounted on a second, reinforcing element having a higher rigidity than the first element.

19. Implantable closure according to claim 18, characterized in that
the first element is made of felt-like material.

20. Implantable closure according to claim 18 or 19, characterized in that the first element and/or second element are absorbable or non-absorbable.

21. Implantable closure according to any one of claims 18 to 20, charcterized in that
the reinforcing element is flexible or non-flexible.

22. Implantable closure according any one of claims 18 to 21, characterized in that
at least one opening is provided extending at least second element.

23. Implantable closure according claim 22, characterized in that
the opening is provided in the first and second elements and extends from the periphery of the closure.

24. Implantable closure according claim 22, characterized in that
the opening is located at a distance form the periphery of the closure.

25. Implantable closure according any one of claims 18 to 24,
further comprising
at least one protrusion protruding from the first element.
